Europäisches Patentamt

European Patent Office

Office européen des brevets

(11) Publication number: **0 227 301 B1**

# EUROPEAN PATENT SPECIFICATION

(45) Date of publication of patent specification: **13.05.92**  (51) Int. Cl.⁵: **C07K 5/06**, C07K 1/02

(21) Application number: **86309057.7**

(22) Date of filing: **19.11.86**

(54) **Process for the preparation of N-formyl-alpha-L-aspartyl-L-phenylalanine methyl ester.**

(30) Priority: **24.12.85 JP 291180/85**

(43) Date of publication of application:
**01.07.87 Bulletin  87/27**

(45) Publication of the grant of the patent:
**13.05.92 Bulletin  92/20**

(84) Designated Contracting States:
**CH GB IT LI NL**

(56) References cited:
**FR-A- 2 249 872**

(73) Proprietor: **AJINOMOTO CO., INC.**
**5-8, Kyobashi 1-chome, Chuo-ku**
**Tokyo 104(JP)**

(72) Inventor: **Yukawa, Toshihide Central Re-
search Laboratories**
**Ajinomoto Co. Inc. 1-1 Suzuki-cho Kawasaki-
ku**
**Kawasaki-shi Kanagawa-ken(JP)**
Inventor: **Kawasaki, Haruo Central Research
Laboratories**
**Ajinomoto Co. Inc. 1-1 Suzuki-cho Kawasaki-
ku**
**Kawasaki-shi Kanagawa-ken(JP)**

Inventor: **Nakamura, Masao Central Research
Laboratories**
**Ajinomoto Co. Inc. 1-1 Suzuki-cho Kawasaki-
ku**
**Kawasaki-shi Kanagawa-ken(JP)**
Inventor: **Yamashita, Takashi**
**Tokai Plant Ajinomoto Co. Inc. No. 1730
Hinaga**
**Yokkaichi-shi Mie-ken(JP)**
Inventor: **Tsuji, Toshiaki**
**Tokai Plant Ajinomoto Co. Inc. No. 1730
Hinaga**
**Yokkaichi-shi Mie-ken(JP)**

(74) Representative: **Bond, Bentley George et al
HASELTINE LAKE & CO. Hazlitt House 28
Southampton Buildings Chancery Lane
London, WC2A 1AT(GB)**

## Description

This invention relates to a process for obtaining N-formyl-α-L-aspartyl-L-phenylalanine methyl ester (hereinafter referred to as PM) and N-formyl-L-aspartic acid anhydride.

α-L-Aspartyl-L-phenylalanine methyl ester (hereinafter referred to as α-APM) is known as an excellent sweetening agent. As a process for its production, there is known a process which comprises subjecting N-formyl-L-aspartic acid anhydride and PM to a condensation reaction in an organic solvent to form N-formyl-α- L-aspartyl-L-phenylalanine methyl ester (hereinafter referred to as For-α-APM) and then removing the formyl group from this reaction product to obtain the desired α-APM (GB-A-2153365).

However in practice, the desired For-α-APM is not the sole product since its isomer, N-formyl-β-L-aspartyl-L-phenylalanine methyl ester (hereinafter referred to as For-β-APM), is also produced, and the ratio of For-α-APM to For-β-APM (hereinafter referred to the α:β ratio) is not always satisfactory, because, even when the practical concentrations of reactants as given in GB-A-2153365 (0.2 to 0.8 M/litre) are employed, the α:β ratio is small (i.e. 1.8:1 to 3.3:1), and therefore the process cannot be regarded as an efficient production process. Indeed, when low concentrations of the reactants according to GB-A-2153365 (0.025 M/litre) are employed, a considerably high α:β ratio of 5.5 is attained. In this case, large reaction vessels are needed. However, such large reactant vessels are not practical from the industrial point of view. On the contrary, according to the present invention, the α:β ratios can be remarkably enhanced at the practical concentrations of the reactants.

The production of For-β-APM as a by-product reduces not only the yield of For-α-APM but also that of the end product, α-APM. This also means that the α-APM is contamined with β-L-aspartyl-L-phenylalanine methyl ester (hereinafter referred to as β-APM). Since β-APM does not exhibit sweetness, α-APM contaminated with β-APM must be purified to remove the β-APM. Therefore, as described above, an increase in the α:β ratio is believed to be very advantageous in order that the desired end product, α-APM, may be obtained in a good yield.

The present inventors have intensively studied in order to overcome this disadvantage and, as a result, have surprisingly discovered that by reacting N-formyl-L-aspartic acid anhydride and PM in a reaction medium in the presence of acetic acid or of a mixture of acetic acid and formic acid in a "complete mixing type continuous reaction mode", the α:β ratio is greatly enhanced, and therefore have accomplished this invention.

Thus, according to the present invention, there is provided a process for the production of N-formyl-α-L-aspartyl-L-phenylalanine methyl ester, characterized in that N-formyl-L-aspartic acid anhydride is reacted with L-phenylalanine methyl ester in a reaction solvent and in the presence of acetic acid or acetic acid and formic acid and in a complete mixing type continuous reaction mode, and in that the amount of acetic acid or acetic acid and formic acid present is from 10 to 150% by weight based on the reaction solvent.

The expression "complete mixing type continuous mode" used herein means that the starting materials (e.g. N-formyl-L-aspartic acid anhydride and PM) are continuously fed to a reaction zone (e.g. a reactor) whilst the mass in the reaction zone (e.g. the mass in the reactor) is being completely mixed (e.g. by stirring) and whilst the resulting reaction mixture is being continuously withdrawn from the reaction zone (e.g. by being allowed to overflow from the reactor).

The N-formyl-L-aspartic acid anhydride used in the process of this invention may be obtained by reacting formic acid and acetic anhydride with L-aspartic acid.

The reaction solvent used in the process of this invention may be any solvent as long as it is not particularly reactive with the reactants and the reaction product. Representative examples thereof include hydrocarbons such as toluene, xylene, hexane, etc.; esters such as ethyl acetate, methyl propionate, etc.; carboxylic acids such as acetic acid, propionic acid, etc.; ketones such as acetone, methyl ethyl ketone, etc.; halogenated hydrocarbons such as chloroform, dichloromethane, ethylene dichloride, etc.; ethers such as diethyl ether, tetrahydrofuran, dioxane, etc.; amides such as dimethylformamide, etc.; dimethylsulfoxide; and water. It is possible to use a mixture comprising two or more solvents.

PM may be obtained by methyl esterifying L-phenylalanine in the presence of an acid catalyst, and in general, it is obtained as an addition salt with said acid, and therefore by neutralizing and subsequently extracting it with one or more of the above-mentioned solvents, it may be directly used in the reaction. The concentration of PM is not particularly restricted. Thus, for example, 0.1 to 0.8 M/litre is employed.

The N-formyl-L-aspartic acid anhydride may be used in the reaction as it is or may be dissolved or suspended in the aforesaid solvent after adding acetic acid or acidic acid and formic acid, and then used in the reaction. The concentration thereof is not particularly restricted. Thus, for example, 0.2 to 4 M/litre is used.

The amount of the acetic acid, or the acetic acid and formic acid, used in the process of this invention is preferably as high as possible in view of the N-formyl-L-aspartic acid anhydride. However, if it is too high, the $\alpha:\beta$ ratio is lowered, and therefore 10 to 150% (by weight) based on the reaction solvent is employed. The formic acid, when used, is preferably used in an amount of not greater than 30% (by weight) based on the acetic acid and not greater than 5% (by weight) based on the reaction solvent.

The molar ratio of N-formyl-L-aspartic anhydride to PM in the process of this invention is not particularly restricted. Thus, for example, 0.5 to 5.0 is employed. The reaction temperature is preferably not higher than 100°C, more preferably not higher than 80°C, so as to prevent racemization of the product.

While there is no particular restriction on the reaction time, the reaction rate between the N-formyl-L-aspartic acid anhydride and the L-phenylalanine methyl ester is large and thus it is not necessary to conduct the reaction for a prolonged time. Generally, depending upon on the reaction temperature, a retention time of within 6 hours is satisfactory. Furthermore, it is also possible to conduct the reaction is a plurality of vessels in several stages (cascade mode).

By this invention, since it is possible to greatly enhance the $\alpha:\beta$ ratio and also greatly improve the yield of the desired end product, $\alpha$-APM and also since the reaction is conducted in a continuous mode, it is efficient also in respect of its apparatus and thus it is extremely advantageous from an industrial point of view.

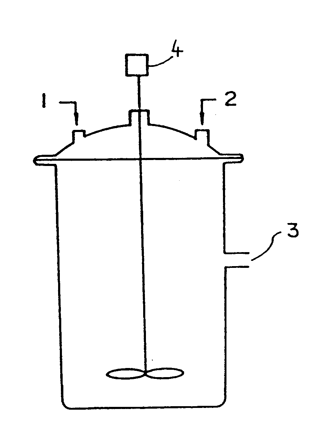

The following Examples illustrate the invention. Reference will be made in the Examples to the single Figure of the drawing , showing a reactor having an inlet 1 for PM solution, and inlet 2 for N-formyl-L-aspartic acid anhydride, an overflow 3, and a stirrer driven by a motor 4.

EXAMPLE 1

To a reactor having an effective capacity of 800 ml, as shown in the Figure, were continuously fed a solution of PM in acetic acid and toluene (acetic acid:toluene = 40:100) in a concentration of 0.5 M/litre, at a rate of 28.6 g/hr, and N-formyl-L-aspartic acid anhydride at a rate of 28.6 g/hr, while the reaction mixture was being completely mixed by stirring, and while the reaction mixture was continuously withdrawn by being allowed to overflow. During this time, the temperature of the reaction mixture was maintained at 30°C. The retention time of the PM and N-formyl-L-aspartic acid anhydride was 120 minutes.

The For-$\alpha$-APM and For-$\beta$-APM in the overflowing reaction mixture were quantitatively determined by HPLC, and their $\alpha:\beta$ ratio was found to be 6.0:1.

EXAMPLE 2

To a reactor having an effective capacity of 800 ml, as shown in Figure, were continuously fed a solution of PM is acetic acid formic acid and toluene (acetic acid:formic acid:toluene = 25:5:1000) in a concentration of 0:5 M/litre at a rate of 400 ml/hr and N-formyl-L-aspartic acid anhydride at a rate of 28.6 g/hr, while the reaction mixture was completely mixed by stirring, and while the reaction mixture was continuously withdrawn by being allowed to overflow. During this time, the temperature of the reaction mixture was maintained at 30°C. The retention time of the N-formyl-L-aspartic acid anhydride was 120 minutes.

The For-$\alpha$-APM and For-$\beta$-APM in the overflowing reaction mixture were quantitively determined by HPLC, and their $\alpha:\beta$ ratio was found to be 5.7:1.

EXAMPLE 3

To a reactor having an effective capacity of 800 ml, as shown in the Figure, were continuously fed a solution of PM in acetic acid and toluene (acetic acid:toluene = 100:100) in a concentration of 0.5 M/litre at a rate of 400 ml/hr, and N-formyl-L-aspartic acid anhydride at a rate of 28.6 g/hr, while the reaction mixture was being completely mixed by stirring, and while the reaction mixture was continuously withdrawn by being allowed to overflow. During this time, the temperature of the reaction mixture was maintained at 15°C. The retention time of the PM and N-formyl-L-aspartic acid anhydride was 120 minutes.

The For-$\alpha$-APM and For-$\beta$-APM in the overflowing reaction mixture were quantitatively determined by HPLC, and their $\alpha:\beta$ ratio was found to be 7.0:1.

EXAMPLE 4

To a reactor having an effective capacity of 800 ml, as shown in the Figure, were continuously fed a solution of PM in acetic acid and toluene (acetic acid:toluene = 20:100) in a concentration of 0.5 M/litre, at a rate of 800 ml/hr, and N-formyl-L-aspartic acid anhydride at a rate of 57.2 g/hr, while the reaction mixture was being completely mixed by stirring, and while the reaction mixture was continuously withdrawn by being allowed to overflow. During this time, the temperature of the reaction mixture was maintained at 30°C. The retention time of the PM and N-formyl-L-aspartic acid anhydride was 120 minutes.

The For-$\alpha$-APM and For-$\beta$-APM in the overflowing reaction mixture were quantitatively determined by HPLC, and their $\alpha$:$\beta$ ratio was found to be 5.7.:1.

EXAMPLE 5

To a reactor having an effective capacity of 800 ml, as shown in the Figure, were continuously fed a solution of PM in acetic acid and toluene (acetic acid:toluene = 40:100) in a concentration of 0.2 M/litre, at a rate of 400 ml/hr, and N-formyl-L-aspartic acid anhydride at a rate of 11.4 g/hr, while the reaction mixture was being completely mixed by stirring, and while the reaction mixture was continuously withdrawn by being allowed to overflow. During this time, the temperature of the reaction mixture was maintained at 30°C. The retention time of the PM and N-formyl-L-aspartic acid anhydride was 120 minutes.

The For-$\alpha$-APM and For-$\beta$-APM in the overflowing reaction mixture were quantitatively determined by HPLC, and their $\alpha$:$\beta$ ratio was found to be 5.7.:1.

EXAMPLE 6

To a reactor having an effective capacity of 800 ml, as shown in the Figure, were continuously fed a solution of PM in acetic acid and butyl acetate (acetic acid:butyl acetate = 40:100) in a concentration of 0.4 M/litre at a rate of 400 ml/hr, and N-formyl-L-aspartic acid anhydride at a rate of 22.9 g/hr, while the reaction mixture was being completely mixed by stirring, and while the reaction mixture was continuously withdrawn by being allowed to overflow. During this time, the temperature of the reaction mixture was maintained at 30°C. The retention time of the PM and N-formyl-L-aspartic acid anhydride was 120 minutes.

The For-$\alpha$-APM and For-$\beta$-APM in the overflowing reaction mixture was quantitatively determined by HPLC, and their $\alpha$:$\beta$ ratio was found to be 5.7.:1.

COMPARATIVE EXAMPLE 1

To a reactor having an effective capacity of 800 ml, as shown in the Figure, were continuously fed a solution of PM in toluene in a concentration of 0.5 M/litre at a rate of 400 ml/hr, and N-formyl-L-aspartic acid anhydride at a rate of 28.6 g/hr, while the reaction mixture was being completely mixed by stirring, and while the reaction mixture was continuously withdrawn by being allowed to overflow. During this time, the temperature of the reaction mixture was maintained at 30°C. The retention time of the PM and N-formyl-L-aspartic acid anhydride was 120 minutes.

The For-$\alpha$-APM and For-$\beta$-APM in the overflowing reaction mixture was quantitatively determined by HPLC, and their $\alpha$:$\beta$ ratio was found to be 0.9:1.

COMPARATIVE EXAMPLE 2

To a reactor having an effective capacity of 800 ml, as shown in the Figure, were continuousy fed a solution of PM in acetic acid and toluene (acetic acid:toluene = 5:100) in a concentration of 0.5 M/litre at a rate of 400 ml/hr, and N-formyl-L-aspartic acid anhydride at a rate of 28.6 g/hr, while the reaction mixture was being completely mixed by stirring, and while the reaction mixture was continuously withdrawn by being allowed to overflow. During this time, the temperature of the reaction mixture was maintained at 30°C. The retention time of the PM and N-formyl-L-aspartic acid anhydride was 120 minutes.

The For-$\alpha$-APM and For-$\beta$-APM in the overflowing reaction mixture were quantitatively determined by HPLC, and their $\alpha$:$\beta$ ratio was found to be 3.7.:1.

COMPARATIVE EXAMPLE 3

To a reactor having an effective capacity of 800 ml, as shown in the Figure, were continuousiy fed a solution of PM in acetic acid and toluene (acetic acid:toluene = 900:100) in a concentration of 0.5 M/litre at a rate of 400 ml/hr, and N-formyl-L-aspartic acid anhydride at a rate of 28.6 g/hr, while the reaction mixture was being completely mixed by stirring, and while the reaction mixture was continuously withdrawn by being allowed to overflow. During this time, the temperature of the reaction mixture was maintained at 30°C. The retention time of the PM and N-formyl-L-aspartic acid anhydride was 120 minutes.

The For-$\alpha$-APM and For-$\beta$-APM in the overflowing reaction mixture were quantitatively determined by HPLC, and their $\alpha$:$\beta$ ratio was found to be 4.3:1.

COMPARATIVE EXAMPLE 4(BATCH REACTION)

7.2 g of an N-formyl-L-aspartic acid anhydride was added to 100 ml of a solution of PM in acetic acid and toluene (acetic acid:toluene = 40:100) in a concentration of 0.5 M/litre, and the mixture was stirred at 30°C for 2 hours.

The For-$\alpha$-APM and For-$\beta$-APM in the overflowing reaction mixture were quantitatively determined by HPLC, and their $\alpha$:$\beta$ ratio was found to be 3.5:1.

## Claims

1. A process for the production of N-formyl-$\alpha$-L-aspartyl-L-phenylalanine methyl ester, characterized in that N-formyl-L-aspartic acid anhydride is reacted with L-phenylalanine methyl ester in a reaction solvent, in the presence of acetic acid or acetic acid and formic acid and in a complete mixing type continuous reaction mode, and in that the amount of acetic acid or acetic acid and formic acid present is from 10 to 150% by weight based on the reaction solvent.

2. A process according to claim 1 wherein the reaction solvent is toluene or ethyl acetate.

## Revendications

1. Procédé de préparation de l'ester méthylique de la N-formyl-$\alpha$-L-aspartyl-L-phénylalanine, caractérisé en ce qu'on fait réagir l'anhydride de l'acide N-formyl-L-aspartique avec l'ester méthylique de la L-phénylalanine dans un solvant réactionnel en présence d'acide acétique ou d'acide acétique et d'acide formique et avec un mode de réaction continu du type mélange complet, et en ce que la quantité d'acide acétique ou d'acide acétique et d'acide formique présente est de 10 à 150 % en poids par rapport au solvant réactionnel.

2. Procédé selon la revendication 1, dans lequel le solvant réactionnel est le toluène ou l'acétate d'éthyle.

## Patentansprüche

1. Verfahren zur Herstellung von N-Formyl-$\alpha$-L-aspartyl-L-phenylalaninmethylester, **dadurch gekennzeichnet,** daß N-Formyl-L-asparaginsäureanhydrid mit L-Phenylalaninmethylester in einem Reaktionslösungsmittel in Gegenwart von Essigsäure oder Essigsäure und Ameisensäure und in einem kontinuierlichen Reaktionsverfahren nach dem Komplettmischungstyp umgesetzt wird, und daß die Menge an Essigsäure oder Essigsäure und Ameisensäure von 10 bis 150 Gew.-%, bezogen auf das Reaktionslösungsmittel, beträgt.

2. Verfahren nach Anspruch 1, worin das Reaktionslösungsmittel Toluol oder Ethylacetat ist.